# EUROPEAN PATENT APPLICATION

(11) **EP 4 785 895 A1**
(43) Date of publication of application: **05.08.2026**
(21) Application number: 24872645.7
(22) Date of filing: 24.05.2024
(51) Int. Cl.: A61B 34/30, A61B 34/20, B25J 9/10, B25J 9/16

(54) **SURGICAL INSTRUMENT CONTROL DEVICE**

(30) Priority: 27.09.2023 KR 20230130306
(71) Applicant: LN Robotics Inc., Seoul 05505 (KR)
(72) Inventor: WON, Jong Seok, Seoul 06226 (KR); CHOI, Seung Hee, Osan-si Gyeonggi-do 18151 (KR); BYUN, Je Ik, Seoul 06000 (KR); PARK, Sang Eun, Seoul 05285 (KR)
(74) Representative: Lavoix
(86) International application number: PCT/KR2024/007074
(87) International publication number: WO 2025/070933

(57) **Abstract**

According to an embodiment, the surgical instrument control device may comprise: a first driving part including a first roller module and a second roller module arranged side by side such that a first surgical instrument is held therebetween; a second driving part including a third roller module and a fourth roller module arranged side by side such that a second surgical instrument is held therebetween; and a processor configured to control operations of the first driving part and the second driving part. The processor may be configured to control the first driving part such that the first surgical instrument moves forward/backward along a path provided by the second surgical instrument in response to a user command that has been input thereto. If the path provided by the second surgical instrument is curved by a movement of the first surgical instrument in a first direction during a process in which the first surgical instrument moves along the path provided by the second surgical instrument, the processor may control the second driving part such that the curvature is alleviated, thereby performing a curvature alleviation procedure in which the second surgical instrument is moved in a second direction opposite to the first direction.

## Description

### TECHNICAL FIELD

The following embodiment relates to a device for controlling a surgical instrument.

### BACKGROUND ART

The existing percutaneous coronary intervention (PCI) procedure imposes a risk of continuous radiation exposure on an operator, requires significant time and cost to train an operator to a level sufficient to perform stable procedures, and exhibits a large gap in procedural quality among operators, regions, and/or hospitals, thereby making it difficult to uniformly provide a high-quality medical service. To compensate for these shortcomings, an intervention procedure assistant robot is introduced. For example, the intervention procedure assistant robot may be configured to move forward, move backward, or rotate a surgical instrument when a user inputs a command.

The above description has been possessed or acquired by the inventor(s) in the course of conceiving the present disclosure and is not necessarily an art publicly known before the present application is filed.

### DISCLOSURE OF THE INVENTION

### TECHNICAL GOALS

An embodiment is to provide a surgical instrument control device that is controlled to mitigate a curvature when the curvature occurs in a path provided by a second surgical instrument as a first surgical instrument moves (e.g., a forward moving motion) in a first direction while the first surgical instrument is moved along the path provided by the second surgical instrument.

An embodiment is to provide a surgical instrument control device that is controlled to mitigate a path difference between a first surgical instrument and a second surgical instrument when the first surgical instrument is bent and moves (e.g., moves forward) in the first direction compared to a path provided by the second surgical instrument due to the resistance applied to an end of the first surgical instrument while the first surgical instrument is moved along the path provided by the second surgical instrument.

### TECHNICAL SOLUTIONS

In an embodiment, a surgical instrument control device includes a first driving part including a first roller module and a second roller module disposed in parallel to grip a first surgical instrument therebetween, a second driving part including a third roller module and a fourth roller module disposed in parallel to grip a second surgical instrument therebetween, and a processor configured to control operations of the first driving part and the second driving part. The processor is further configured to control the first driving part to move the first surgical instrument forward and backward along a path provided by the second surgical instrument in response to an input user command, and while the first surgical instrument is moved along the path provided by the second surgical instrument, when a curvature occurs in the path provided by the second surgical instrument as the first surgical instrument is moved in a first direction, perform a curvature mitigating procedure to move the second surgical instrument in a second direction that is opposite to the first direction to mitigate the curvature.

In an embodiment, the processor is further configured to perform the curvature mitigating procedure by controlling the second driving part.

In an embodiment, positions or shapes of the first surgical instrument and the second surgical instrument are read using an angiogram.

In an embodiment, the processor is further configured to determine whether a curvature occurs in the path provided by the second surgical instrument by analyzing the angiogram.

In an embodiment, when the curvature is determined to be mitigated by a reference value or more in the path provided by the second surgical instrument, the processor is further configured to stop the curvature mitigating procedure.

In an embodiment, the first surgical instrument is moved along the path provided by the second surgical instrument while surrounding the second surgical instrument from an outside.

In an embodiment, the first surgical instrument includes one of a balloon catheter, a stent catheter, or a microcatheter. The second surgical instrument includes a guide wire.

In an embodiment, a surgical instrument control device includes a first driving part including a first roller module and a second roller module disposed in parallel to grip a first surgical instrument therebetween, a second driving part including a third roller module and a fourth roller module disposed in parallel to grip a second surgical instrument therebetween, and a processor configured to control operations of the first driving part and the second driving part. The processor is further configured to control the first driving part to move the first surgical instrument forward and backward along a path provided by the second surgical instrument in response to an input user command, and while the first surgical instrument is moved along the path provided by the second surgical instrument, when an end portion of the first surgical instrument is not moved in a first direction due to resistance applied to the end portion of the first surgical instrument, perform a path difference mitigating procedure to move the second surgical instrument in the first direction to mitigate a path difference between the first surgical instrument and the second surgical instrument.

In an embodiment, the processor is further configured to perform the path difference mitigating procedure by controlling the second driving part.

In an embodiment, an end portion of the second surgical instrument is disposed across an entrance of a blood vessel leading to a lesion.

In an embodiment, positions or shapes of the first surgical instrument and the second surgical instrument are read using an angiogram.

In an embodiment, the processor is further configured to analyze the angiogram to determine whether the end portion of the first surgical instrument is not moved in the first direction while a force to move the first surgical instrument is applied.

In an embodiment, when the path difference between the first surgical instrument and the second surgical instrument is determined to be mitigated by a reference value or more, the processor is further configured to stop the path difference mitigating procedure.

In an embodiment, the first surgical instrument is moved inside the second surgical instrument along the path provided by the second surgical instrument.

In an embodiment, the first surgical instrument includes one of a guide wire or a microcatheter. The second surgical instrument includes a guide catheter.

### EFFECTS OF THE INVENTION

A surgical instrument control device according to an embodiment may be controlled to mitigate a curvature that may occur in a second surgical instrument while the first surgical instrument is moved along a path provided by the second surgical instrument, thereby more stably moving the first surgical instrument along the path provided by the second surgical instrument.

A surgical instrument control device according to an embodiment may be controlled to mitigate a path difference that may occur between a first surgical instrument and a second surgical instrument while the first surgical instrument is moved along a path provided by the second surgical instrument, thereby more stably moving the first surgical instrument along the path provided by the second surgical instrument.

The effects of the surgical instrument control device are not limited to the above-mentioned effects, and other unmentioned effects can be clearly understood from the above description by those having ordinary skill in the technical field to which the present disclosure pertains.

### BRIEF DESCRIPTION OF DRAWINGS

FIG. 1 is a perspective view of a surgical instrument control device according to an embodiment.
FIG. 2 is a plan view of a surgical instrument control device according to an embodiment.
FIG. 3 is an example of a usage state view of a surgical instrument control device according to an embodiment.
FIG. 4 is a front view of a portion of a roller module of a surgical instrument control device according to an embodiment.
FIGS. 5A to 5C are cross-sectional views of a process in which a first surgical instrument is inserted along a path provided by a second surgical instrument according to an embodiment.
FIGS. 6A to 6C are cross-sectional views of a process in which a first surgical instrument is inserted along a path provided by a second surgical instrument according to an embodiment.

### BEST MODE FOR CARRYING OUT THE INVENTION

Hereinafter, embodiments are described in detail with reference to the accompanying drawings. However, various alterations and modifications may be made to the embodiments. Here, the embodiments are not construed as limited to the disclosure. The embodiments should be understood to include all changes, equivalents, and replacements within the technical scope of the disclosure.

The terminology used herein is for the purpose of describing particular embodiments only and is not to be limiting of the embodiments. As used herein, the singular form is intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms including technical and scientific terms used herein have the same meaning as commonly understood by one of ordinary skill in the art to which the examples belong. Terms, such as those defined in commonly used dictionaries, are to be interpreted as having a meaning that is consistent with their meaning in the context of the relevant art and the present disclosure, and are not to be interpreted in an idealized or overly formal sense unless expressly so defined herein.

When describing the embodiments with reference to the accompanying drawings, like reference numerals refer to like components regardless of drawing numbers and a repeated description related thereto will be omitted. In the description of embodiments, detailed description of well-known related technology will be omitted when it is deemed that such description will cause ambiguous interpretation of the present disclosure.

In addition, the terms first, second, A, B, (a), and (b) may be used to describe components of the embodiments. These terms are used only for the purpose of discriminating one component from another component, and the nature, the sequences, or the orders of the components are not limited by the terms. It should be noted that if one component is described as being "connected," "coupled" or "joined" to another component, the former may be directly "connected," "coupled," and "joined" to the latter or "connected", "coupled", and "joined" to the latter via another component.

The same name may be used to describe an element included in the embodiments described above and an element having a common function. Unless otherwise mentioned, the descriptions on the embodiments may be applicable to the following embodiments and thus, duplicated descriptions will be omitted for conciseness.

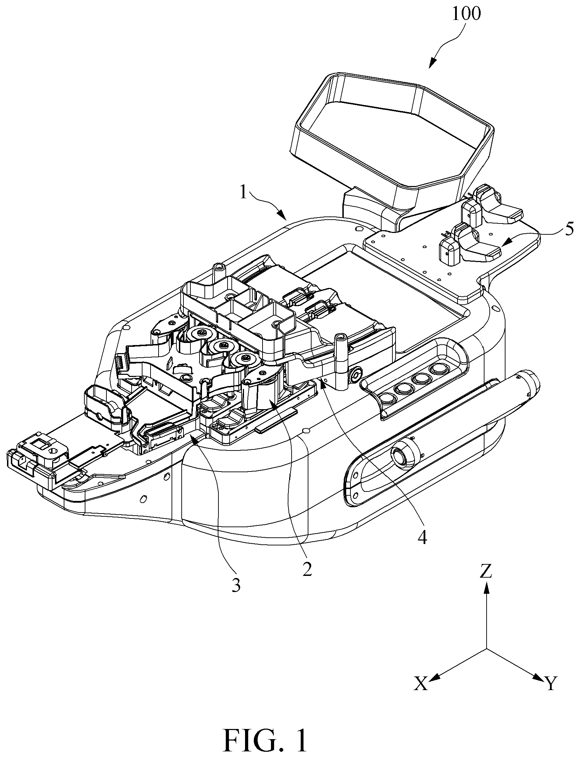

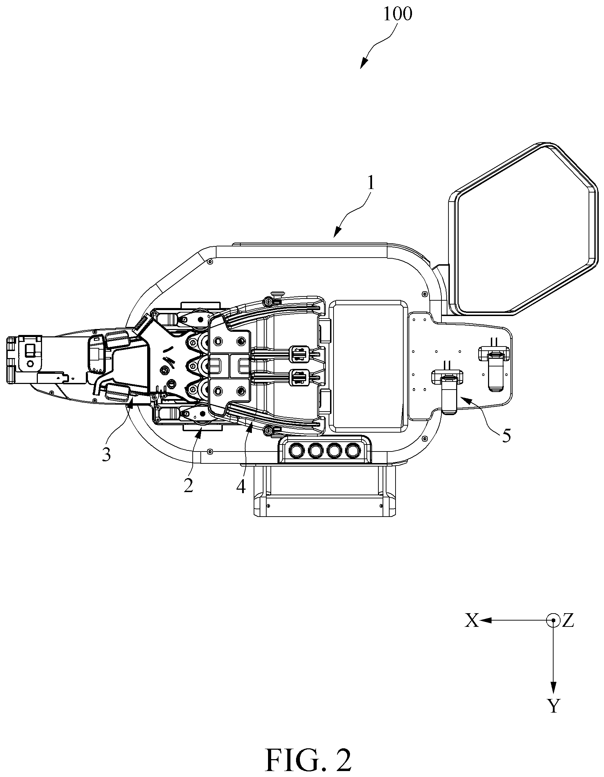

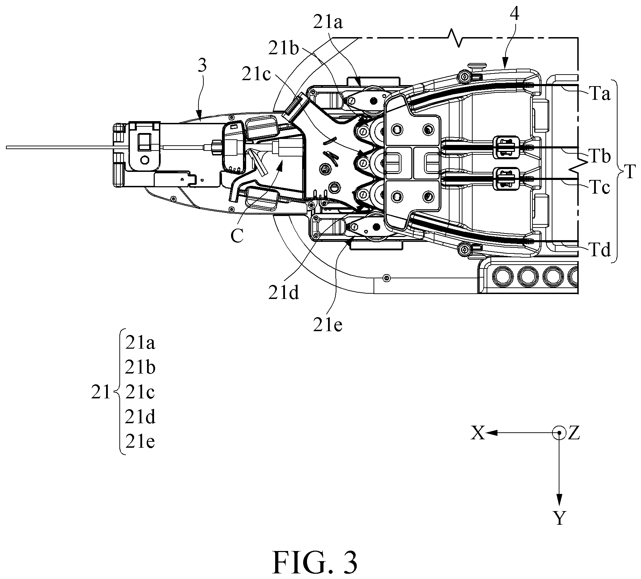

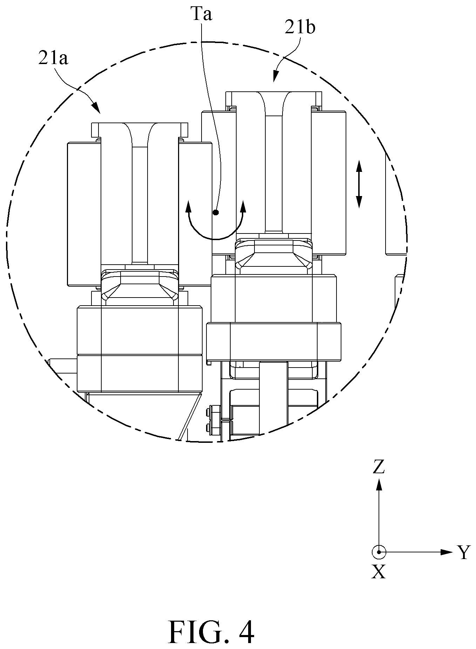

FIG. 1 is a perspective view of a surgical instrument control device according to an embodiment. FIG. 2 is a plan view of a surgical instrument control device according to an embodiment. FIG. 3 is an example of a usage state view of a surgical instrument control device according to an embodiment. FIG. 4 is a front view of a portion of a roller module of a surgical instrument control device according to an embodiment.

Referring to FIGS. 1 to 4, a surgical instrument control device 100 may independently control at least one surgical instrument T. The surgical instrument control device 100 may grip or release the surgical instrument T between roller modules 21 described below by horizontal (e.g., the y direction) movement of the roller module 21. The surgical instrument control device 100 may implement forward/backward movement and/or rotation of the surgical instrument T by rotation and/or vertical (e.g., the z direction) movement of the roller module 21. The surgical instrument control device 100 may be used for, for example, percutaneous coronary intervention (PCI). However, this is an example, and the purpose of the surgical instrument control device 100 is not limited thereto. The surgical instrument T may refer to a surgical instrument having a longitudinal direction. For example, the surgical instrument T may include at least one of a guide wire, a balloon catheter, and a guide catheter having a longitudinal direction. However, this is an example, and the type of the surgical instrument T is not limited thereto.

Hereinafter, in describing the surgical instrument control device 100, "distal" may be construed as a side in a direction toward a treatment site (e.g., the side in the +x direction), and "proximal" may be construed as a side in a direction away from the treatment site (e.g., the side in the -x direction).

The surgical instrument control device 100 according to an embodiment may include a housing 1, a driving assembly 2, a connector mounter 3, a surgical instrument proximal guide 4, a rear holder 5, and a processor (not shown).

In an embodiment, the housing 1 may form an exterior of the surgical instrument control device 100. The housing 1 may provide an area where the driving assembly 2, the connector mounter 3, the surgical instrument proximal guide 4, and/or the rear holder 5 are supported. For example, the housing 1 may be connected to a robotic arm of a slave base. The surgical instrument control device 100 may function as an end effector connected to the robotic arm of the slave base. In the housing 1, a handle and/or a switch for adjusting a position of the housing 1 and/or tilting the housing 1 may be provided. In the housing 1, a display panel for conveying information on a state of the device and/or how to use the device to the user may be provided.

In an embodiment, the driving assembly 2 may grip or release the surgical instrument T. The driving assembly 2 may move or rotate the gripped surgical instrument T in a longitudinal direction.

In an embodiment, the driving assembly 2 may include a plurality of roller modules 21. At least a pair of roller modules 21 may be provided. For example, as shown in the drawing, the roller module 21 may include a first roller module 21a, a second roller module 21b, a third roller module 21c, a fourth roller module 21d, and a fifth roller module 21e. The plurality of roller modules 21 may be arranged parallel to each other. For example, the driving assembly 2 may independently control four surgical instruments Ta, Tb, Tc, and Td by five roller modules 21a, 21b, 21c, 21d, and 21e. However, this is an example, and the number of roller modules 21 is not limited thereto.

In an embodiment, the surgical instrument T may be gripped between two adjacent roller modules 21. For this, at least one roller module 21 may horizontally move toward the other roller module 21. For example, the second roller module 21b may horizontally move toward the first roller module 21a such that the first surgical instrument Ta is gripped between the first roller module 21a and the second roller module 21b. In this case, the second surgical instrument Tb positioned between the second roller module 21b and the third roller module 21c may be released. In addition, on the other hand, the second roller module 21b may horizontally move toward the third roller module 21c such that the second surgical instrument Tb is gripped between the second roller module 21b and the third roller module 21c. In this case, the first surgical instrument Ta positioned between the first roller module 21a and the second roller module 21b may be released.

Similarly, the fourth roller module 21d may horizontally move toward the third roller module 21c such that the third surgical instrument Tc is gripped between the third roller module 21c and the fourth roller module 21d. In this case, the fourth surgical instrument Td positioned between the fourth roller module 21d and the fifth roller module 21e may be released. In addition, on the other hand, the fourth roller module 21d may horizontally move toward the fifth roller module 21e such that the fourth surgical instrument Td is gripped between the fourth roller module 21d and the fifth roller module 21e. In this case, the third surgical instrument Tc positioned between the third roller module 21c and the fourth roller module 21d may be released.

In an embodiment, the driving assembly 2 may implement forward/backward movement and/or rotation of the surgical instrument T by rotation and/or vertical movement of the roller module 21. For example, while the first roller module 21a and the second roller module 21b are positioned adjacent to each other and grip the first surgical instrument Ta therebetween, the gripped first surgical instrument Ta may move forward or backward in the longitudinal direction by rotating the first roller module 21a and the second roller module 21b in one direction or other directions. In addition, as shown in FIG. 4, while the first roller module 21a and the second roller module 21b are positioned adjacent to each other and grip the first surgical instrument Ta therebetween, the gripped first surgical instrument Ta may rotate by moving at least one of the roller modules 21a and 21b in the vertical direction.

In an embodiment, the driving assembly 2 may include at least one driving part. The driving part may be a part for controlling a surgical instrument and may include a pair of roller modules adjacent to each other. For example, the driving part may include a pair of roller modules for controlling a surgical instrument. For example, the driving assembly 2 may include a first driving part and a second driving part. For example, the first driving part may include the first roller module 21a and the second roller module 21b disposed in parallel to grip the first surgical instrument Ta therebetween. For example, the second driving part may include the third roller module 21c and the fourth roller module 21d disposed in parallel to grip the second surgical instrument Tb therebetween. However, this is an example, and the driving part may be formed in various combinations depending on a horizontal position of the roller module 21.

In an embodiment, a processor (not shown) may be configured to control an operation of the driving assembly 2. For example, the processor may be configured to control rotation and/or vertical movement of the roller module 21 to implement forward movement and/or rotation of the surgical instrument T. For example, the processor may be configured to control operations of the first driving part and the second driving part. For example, the processor may be configured to control the driving assembly 2 in response to a user command. For example, the processor may be configured to perform an arbitrary procedure (e.g., a curvature mitigating procedure or a path difference mitigating procedure) to enable smooth movement of the surgical instrument T.

In an embodiment, the connector mounter 3 may be connected to the housing 1 such that the connector mounter 3 is positioned distal (e.g., a side in the +x direction) of the housing 1. The connector mounter 3 may be attached to and detached from the housing 1. The connector mounter 3 may be a consumable and replaceable. The connector mounter 3 may include, for example, a polycarbonate material. However, this is an example, and the material of connector mounter 3 is not limited thereto.

In an embodiment, the connector mounter 3 may hold a surgical instrument connector C. For example, the surgical instrument connector C may be a component configured to collect at least one surgical instrument T and guide a path of the at least one surgical instrument T. For example, the surgical instrument connector C may include a Y connector. However, this is an example, and the type of the surgical instrument connector C is not limited thereto. The connector mounter 3 may guide a path of the surgical instrument T passing through the surgical instrument connector C.

In an embodiment, the surgical instrument proximal guide 4 may be connected to the housing 1 such that the surgical instrument proximal guide 4 is positioned proximal (e.g., the side in the -x direction) compared to the connector mounter 3. The surgical instrument proximal guide 4 may be attached to and detached from the housing 1. The surgical instrument proximal guide 4 may be a consumable and replaceable. The surgical instrument proximal guide 4 may include, for example, a polycarbonate material. However, this is an example, and the material of the surgical instrument proximal guide 4 is not limited thereto.

In an embodiment, the surgical instrument proximal guide 4 may guide a path of the surgical instrument T gripped by the driving assembly 2. For example, the surgical instrument proximal guide 4 may be connected to the housing 1 to cover at least a portion (e.g., a proximal portion) of the driving assembly 2. The surgical instrument proximal guide 4 may include at least one channel to insert and dispose the surgical instrument T therein. Each channel may be formed between two adjacent roller modules 21 of the driving assembly 2.

In an embodiment, the rear holder 5 may be connected to the housing 1 such that the rear holder 5 is positioned proximal (e.g., the side in the -x direction) of the housing 1. The rear holder 5 may hold a position of the surgical instrument T. For example, the rear holder 5 may include a clamp structure. For example, one rear holder 5 or a plurality of rear holders 5 may be provided. The rear holder 5 may be a consumable and replaceable. The rear holder 5 may include, for example, a polycarbonate material. However, this is an example, and the material of the rear holder 5 is not limited thereto.

In an embodiment, the connector mounter 3, the surgical instrument proximal guide 4, and/or the rear holder 5 may be sealed after sterilization and may be provided as disposable products. After the connector mounter 3, the surgical instrument proximal guide 4, and/or the rear holder 5 are opened on site for each procedure and are coupled to the housing 1, the connector mounter 3, the surgical instrument proximal guide 4, and/or the rear holder 5 may hold the plurality of surgical instruments T or may guide the path. When the procedure is terminated, the connector mounter 3, the surgical instrument proximal guide 4, and/or the rear holder 5 may be removed from the housing 1 and may be discarded.

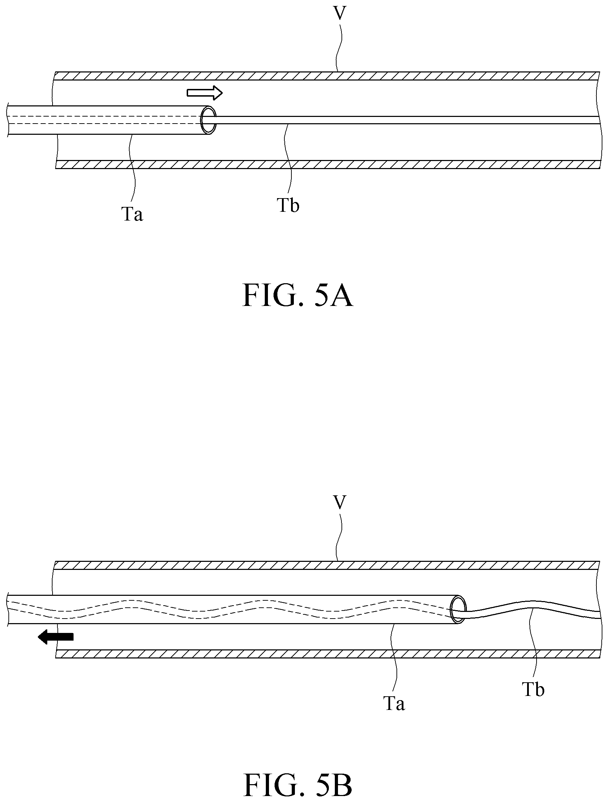

FIGS. 5A to 5C are cross-sectional views of a process in which a first surgical instrument is inserted along a path provided by a second surgical instrument according to an embodiment. FIGS. 5A to 5C are construed as a process of moving the first surgical instrument Ta along a path provided by the second surgical instrument Tb while the second surgical instrument Tb is inserted into and is positioned in a blood vessel V of a patient. For example, the first surgical instrument Ta may include one of a balloon catheter, a stent catheter, or a microcatheter, and the second surgical instrument Tb may include a guide wire. However, this is an example, and the type of the first surgical instrument Ta and/or the second surgical instrument Tb is not limited thereto.

Hereinafter, with reference to FIGS. 5A to 5C, a curvature mitigating procedure of a surgical instrument control device (e.g., the surgical instrument control device 100 of FIG. 1) is described.

In an embodiment, in response to an input user command, a processor may be configured to control a first driving part such that the first surgical instrument Ta moves forward or backward along a path provided by the second surgical instrument Tb. The first driving part may refer to a pair of roller modules gripping the first surgical instrument Ta. For example, when the first surgical instrument Ta is gripped between the first roller module 21a and the second roller module 21b of FIG. 3, the first driving part may include the first roller module 21a and the second roller module 21b. The processor may move the first surgical instrument Ta forward and backward by rotating at least one of the first roller module 21a and the second roller module 21b.

In an embodiment, the first surgical instrument Ta may move along the path provided by the second surgical instrument Tb while surrounding the second surgical instrument Tb from the outside. For example, as illustrated by the white arrow in FIG. 5A, the first surgical instrument Ta may move (e.g., move forward) in a first direction along the path provided by the second surgical instrument Tb. While the first surgical instrument Ta moves along the path provided by the second surgical instrument Tb, a curvature may occur in the path provided by the second surgical instrument Tb due to the movement of the first surgical instrument Ta in the first direction (e.g., a forward motion). For example, when the first surgical instrument Ta moves (e.g., moves forward) in the first direction, the second surgical instrument Tb may move (e.g., move forward) in the first direction unintentionally by a partial length together with the first surgical instrument Ta due to a frictional force between the first surgical instrument Ta and the second surgical instrument Tb. When the second surgical instrument Tb moves (e.g., moves forward) in the first direction by the partial length, an excess length may be generated in the second surgical instrument Tb, and as shown in FIG. 5B, a curvature may occur in at least a portion of the second surgical instrument Tb. For example, the occurrence of curvature in at least a portion of the second surgical instrument Tb may be construed as indicating that an unintended curvature has occurred in the second surgical instrument Tb. For example, the occurrence of curvature in at least a portion of the second surgical instrument Tb may indicate that a path of at least a portion of the second surgical instrument Tb is deformed, the path is bent, the path is wrinkled, or the path is tortuous. When the curvature occurs in the second surgical instrument Tb, moving the first surgical instrument Ta along the path provided by the second surgical instrument Tb may be restricted. For example, the first surgical instrument Ta may not smoothly move at the curvature portion in the second surgical instrument Tb. Alternatively, the body of the patient may be damaged by the curvature of the second surgical instrument Tb.

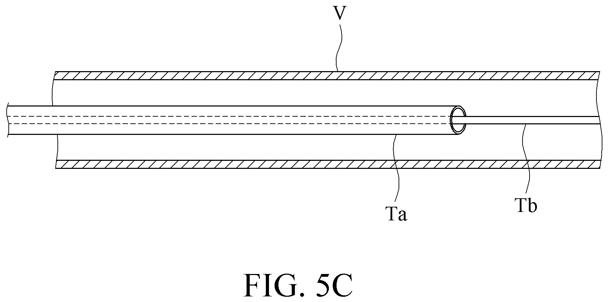

In an embodiment, in the situation described above, the processor may perform a curvature mitigating procedure to mitigate the curvature occurring in the second surgical instrument Tb. For example, the curvature mitigating procedure may include an operation to move (e.g., move backward) the second surgical instrument Tb in a second direction that is opposite to the first direction by controlling the second driving part. The second driving part may refer to a pair of roller modules gripping the second surgical instrument Tb. For example, when the second surgical instrument Tb is gripped between the third roller module 21c and the fourth roller module 21d of FIG. 3, the first driving part may include the third roller module 21c and the fourth roller module 21d. To perform the curvature mitigating procedure, the processor may move (e.g., move backward) the second surgical instrument Tb in the second direction that is opposite to the first direction by rotating at least one of the third roller module 21c and the fourth roller module 21d. For example, as shown in the black arrow in FIG. 5B, by the curvature mitigating procedure of the processor, the second surgical instrument Tb may move (e.g., move backward) in the second direction that is opposite to the first direction relative to the first surgical instrument Ta. For example, when the second surgical instrument Tb moves backward, tension may be applied to the second surgical instrument Tb, thereby mitigating the curvature occurring in the second surgical instrument Tb as shown in FIG. 5C. Meanwhile, the expression that curvature is mitigated may include that the curvature is completely removed. In addition, it is described that the processor performs the curvature mitigating procedure by controlling the second driving part. However, this is an example, and the processor may also perform the curvature mitigating procedure by controlling a separate driving part configured to drive the second surgical instrument.

In an embodiment, the processor may be configured to determine whether a curvature has occurred in the path provided by the second surgical instrument Tb by analyzing an angiogram. For example, positions or shapes of the first surgical instrument Ta and the second surgical instrument Tb may be read using the angiogram. For example, the processor may determine whether an unintended curvature has occurred in the path provided by the second surgical instrument Tb by analyzing an image of the angiogram. For example, the analysis of an angiographic image may be trained via machine learning or may be performed by artificial intelligence. For example, the processor may include a database related to a process of inserting the first surgical instrument Ta and the second surgical instrument Tb and may control the first surgical instrument Ta and/or the second surgical instrument Tb in a feed-forward manner based on the database. When the processor determines that the curvature has occurred in the path provided by the second surgical instrument Tb, the processor may immediately or automatically perform the curvature mitigating procedure. When the processor determines that the curvature occurring in the path provided by the second surgical instrument Tb is mitigated by a reference value or more, the processor may stop the curvature mitigating procedure. However, this is an example, and the curvature mitigating procedure may be performed and/or stopped by a user command. For example, a user may determine whether the curvature has occurred in the path provided by the second surgical instrument Tb via the angiogram and may input a command to perform the curvature mitigating procedure. In an embodiment, a surgical instrument control device (e.g., the surgical instrument control device 100 of FIG. 1) may further include a sensor configured to determine whether a curvature has occurred in the path provided by the second surgical instrument Tb.

In an embodiment, according to the curvature mitigating procedure described above, while moving the first surgical instrument Ta along the path provided by the second surgical instrument Tb, the curvature that may occur in the second surgical instrument Tb may be immediately or automatically mitigated, thereby more stably moving the first surgical instrument Ta along the path provided by the second surgical instrument Tb and reducing damage to the body of a patient.

However, the situations described with reference to FIGS. 5A to 5C are examples, and the curvature mitigating procedure is not performed only in the situations described with reference to FIGS. 5A to 5C. For example, in an embodiment, even when a curvature has occurred in a path of the second surgical instrument Tb while the first surgical instrument Ta is moved inside the second surgical instrument Tb, the curvature mitigating procedure may be performed. In addition, the situations described with reference to FIGS. 5A to 5C are examples, and when a curvature has occurred in a path provided by the second surgical instrument while the first surgical instrument moves backward, the curvature mitigating procedure to move the second surgical instrument forward may be performed.

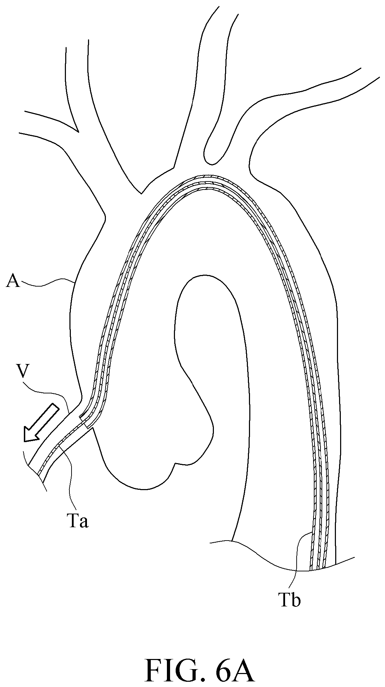

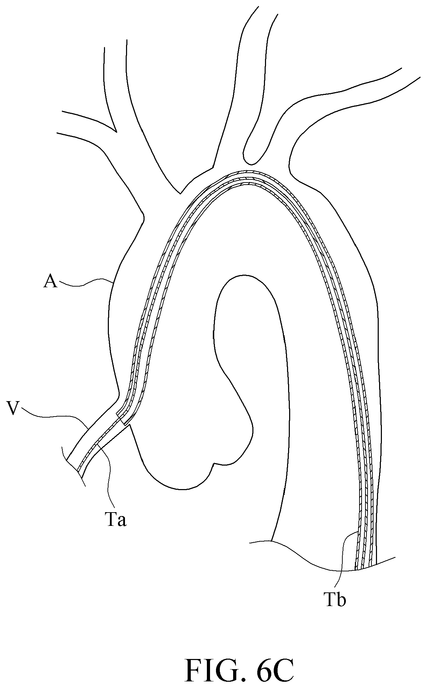

FIGS. 6A to 6C are cross-sectional views of a process in which a first surgical instrument is inserted along a path provided by a second surgical instrument according to an embodiment. FIGS. 6A to 6C may be construed as a process of moving the first surgical instrument Ta along a path provided by the second surgical instrument Tb while an end portion of the second surgical instrument Tb is positioned in an aorta A such that the end portion of the second surgical instrument Tb is across an entrance of a blood vessel (e.g., an entrance of a coronary artery V) leading to a lesion. For example, the first surgical instrument Ta may include one of a guide wire or a microcatheter, and the second surgical instrument Tb may include a guide catheter. However, this is an example, and the type of the first surgical instrument Ta and/or the second surgical instrument Tb is not limited thereto.

Hereinafter, with reference to FIGS. 6A to 6C, a path difference mitigating procedure of a surgical instrument control device (e.g., the surgical instrument control device 100 of FIG. 1) is described.

In an embodiment, in response to an input user command, a processor may be configured to control a first driving part such that the first surgical instrument Ta moves forward or backward along a path provided by the second surgical instrument Tb. The first driving part may refer to a pair of roller modules gripping the first surgical instrument Ta. For example, when the first surgical instrument Ta is gripped between the first roller module 21a and the second roller module 21b of FIG. 3, the first driving part may include the first roller module 21a and the second roller module 21b. The processor may move the first surgical instrument Ta forward and backward by rotating at least one of the first roller module 21a and the second roller module 21b.

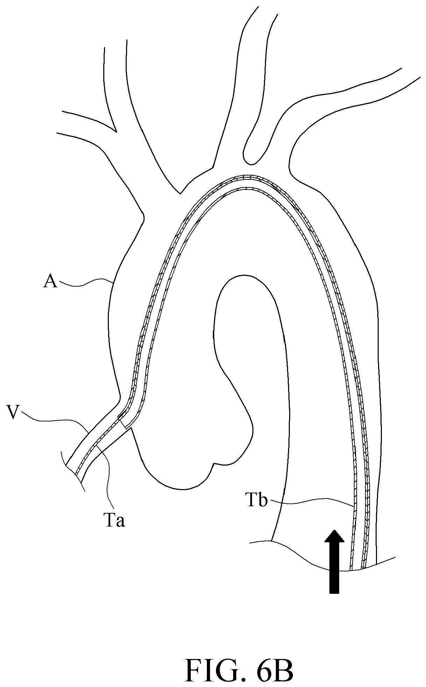

In an embodiment, inside the second surgical instrument Tb, the first surgical instrument Ta may move along a path provided by the second surgical instrument Tb. For example, as illustrated by the white arrow in FIG. 6A, the first surgical instrument Ta may move (e.g., move forward) in a first direction along the path provided by the second surgical instrument Tb. Meanwhile, while the first surgical instrument Ta is moved, resistance may be applied to an end portion of the first surgical instrument Ta. For example, when the first surgical instrument Ta is moved (e.g., is moved forward) in the first direction to pass through a lesion site in the blood vessel, resistance may be applied to the end portion of the first surgical instrument Ta in a direction that is different from the first direction (e.g., the forward direction) due to the blocked lesion site. For example, when the first surgical instrument Ta is moved (e.g., is moved forward) in the first direction during a process of entering the blood vessel with the first surgical instrument Ta, the resistance may be applied to the end portion of the first surgical instrument Ta in a direction that is different from the first direction (e.g., the forward direction). As described above, when the first surgical instrument Ta is moved (e.g., is moved forward) in the first direction while resistance is applied to the end portion of the first surgical instrument Ta, the end portion of the first surgical instrument Ta may no longer move (e.g., move forward) in the first direction. For example, when a force to move (e.g., move forward) the first surgical instrument Ta in the first direction is applied to the end portion of the first surgical instrument Ta?? while the resistance is applied to the end portion of the first surgical instrument Ta, the first surgical instrument Ta may be substantially bent in the second surgical instrument Tb as shown in FIG. 6B. Accordingly, the length of the first surgical instrument Ta inside the second surgical instrument Tb in a state shown in FIG. 6B may be longer than the length of the first surgical instrument Ta inside the second surgical instrument Tb in a state shown in FIG. 6A. When the first surgical instrument Ta becomes longer inside the second surgical instrument Tb, the length of the first surgical instrument Ta may be longer than the path provided by the second surgical instrument Tb. In other words, a path difference between the first surgical instrument Ta and the second surgical instrument Tb may occur. In this state, since the first surgical instrument Ta is positioned longer in the second surgical instrument Tb, an elastic force and/or tension of the first surgical instrument Ta may be applied to the second surgical instrument Tb. When the force is applied to the second surgical instrument Tb, an end portion of the second surgical instrument Tb may be deviated from the entrance of the coronary artery V, the body of a patient may be damaged as the second surgical instrument Tb is deviated, or the treatment may be delayed by repeating a process of reverting the position of the second surgical instrument Tb that is deviated.

In an embodiment, in the situation described above, the processor may perform a path difference mitigating procedure to mitigate the path difference between the first surgical instrument Ta and the second surgical instrument Tb. For example, the path difference mitigating procedure may include an operation of moving (e.g., moving forward) the second surgical instrument Tb in the first direction by controlling the second driving part. The second driving part may refer to a pair of roller modules gripping the second surgical instrument Tb. For example, when the second surgical instrument Tb is gripped between the third roller module 21c and the fourth roller module 21d of FIG. 3, the first driving part may include the third roller module 21c and the fourth roller module 21d. To perform the curvature mitigating procedure, the processor may move (e.g., move forward) the second surgical instrument Tb in the first direction by rotating at least one of the third roller module 21c and the fourth roller module 21d. For example, as shown in the black arrow in FIG. 6B, by the path difference mitigating procedure of the processor, the second surgical instrument Tb may move (e.g., move forward) in the first direction relative to the first surgical instrument Ta. For example, when the second surgical instrument Tb moves (e.g., moves forward) in the first direction, the path difference between the first surgical instrument Ta and the second surgical instrument Tb may be mitigated, thereby mitigating the length difference therebetween. Meanwhile, the expression that the path difference is mitigated may be construed as indicating that the path of the first surgical instrument Ta becomes substantially the same as or similar to the path of the second surgical instrument Tb. In addition, it is described that the processor performs the path difference mitigating procedure by controlling the second driving part. However, this is an example, and the processor may also perform the path difference mitigating procedure by controlling a separate driving part configured to drive the second surgical instrument.

In an embodiment, by analyzing an angiogram, the processor may be configured to determine whether the end portion of the first surgical instrument Ta no longer moves (e.g., moves forward) in the first direction while a force to move the first surgical instrument Ta in the first direction is applied. For example, positions or shapes of the first surgical instrument Ta and the second surgical instrument Tb may be read using the angiogram. For example, by analyzing an image of the angiogram, the processor may determine whether the end portion of the first surgical instrument Ta is not moved (e.g., moved forward) in the first direction while the force to move (e.g., move forward) the first surgical instrument Ta in the first direction is applied. Alternatively, the processor may determine whether a curvature has occurred in the first surgical instrument Ta compared to the path provided by the second surgical instrument Tb by analyzing the image of the angiogram. For example, the processor may determine whether the path difference between the first surgical instrument Ta and the second surgical instrument Tb has occurred by analyzing the image of the angiogram. For example, the analysis of an angiographic image may be trained via machine learning or may be performed by artificial intelligence. For example, the processor may include a database related to a process of inserting the first surgical instrument Ta and the second surgical instrument Tb and may control the first surgical instrument Ta and/or the second surgical instrument Tb in a feed-forward manner based on the database. When the processor determines that the end portion of the first surgical instrument Ta is not moved (e.g., moved forward) in the first direction while the force to move (e.g., move forward) the first surgical instrument Ta in the first direction is applied (in other words, when the processor determines that the path difference between the first surgical instrument Ta and the second surgical instrument Tb has occurred), the processor may immediately or automatically perform the path difference mitigating procedure. When the processor determines that the end portion of the first surgical instrument Ta moves (e.g., moves forward) in the first direction, the processor may stop the path difference mitigating procedure. Alternatively, when the processor determines that the path difference between the first surgical instrument Ta and the second surgical instrument Tb is mitigated by a reference value or more, the processor may stop the path difference mitigating procedure. However, this is an example, and the path difference mitigating procedure may be performed and/or be stopped by a user command. For example, by using the angiogram, a user may determine whether the end portion of the first surgical instrument Ta is not moved (e.g., moved forward) in the first direction while the force to move (e.g. move forward) the first surgical instrument Ta in the first direction is applied and/or whether the path difference between the first surgical instrument Ta and the second surgical instrument Tb has occurred, and the user may input a command to perform the path difference mitigating procedure. In an embodiment, the surgical instrument control device (e.g., the surgical instrument control device 100 of FIG. 1) may further include a sensor configured to measure a distance that the first surgical instrument Ta and/or the second surgical instrument Tb travels. In an embodiment, the surgical instrument control device (e.g., the surgical instrument control device 100 of FIG. 1) may further include a sensor configured to measure a position of the end portion of the first surgical instrument Ta. In an embodiment, the surgical instrument control device (e.g., the surgical instrument control device 100 of FIG. 1) may further include a sensor configured to determine whether a curvature has occurred in the first surgical instrument Ta compared to the path provided by the second surgical instrument Tb (in other words, when a path difference between the first surgical instrument Ta and the second surgical instrument Tb is determined to have occurred).

In an embodiment, according to the path difference mitigating procedure described above, while moving the first surgical instrument Ta along the path provided by the second surgical instrument Tb, the path difference that may occur between the first surgical instrument Ta and the second surgical instrument Tb may be immediately or automatically mitigated, thereby more stably moving the first surgical instrument Ta along the path provided by the second surgical instrument Tb and reducing damage to the body of a patient.

However, the situations described with reference to FIGS. 6A to 6C are examples, and the path difference mitigating procedure is not performed only in the situations described with reference to FIGS. 6A to 6C. For example, in an embodiment, when the path difference between the first surgical instrument Ta and the second surgical instrument Tb has occurred while the first surgical instrument Ta surrounds the outside of the second surgical instrument Tb and is moved, the path difference mitigating procedure may be performed. In addition, the situations described with reference to FIGS. 6A to 6C are examples, and when a path difference between the first surgical instrument and the second surgical instrument has occurred while the first surgical instrument moves backward, the path difference mitigating procedure to move the second surgical instrument backward may be performed.

Although the embodiments are described with reference to the limited drawings, one of ordinary skill in the art may apply various technical modifications and variations based thereon. For example, suitable results may be achieved if the described techniques are performed in a different order, and/or if components in a described system, structure, device, or circuit are combined in a different manner, and/or replaced or supplemented by other components or their equivalents.

Therefore, other implementations, other embodiments, and equivalents to the claims are also within the scope of the following claims.

## Claims

1. A surgical instrument control device comprising:
a first driving part comprising a first roller module and a second roller module disposed in parallel to grip a first surgical instrument therebetween;
a second driving part comprising a third roller module and a fourth roller module disposed in parallel to grip a second surgical instrument therebetween; and
a processor configured to control operations of the first driving part and the second driving part,
wherein the processor is further configured to:
control the first driving part to move the first surgical instrument forward and backward along a path provided by the second surgical instrument in response to an input user command, and
while the first surgical instrument is moved along the path provided by the second surgical instrument, when a curvature occurs in the path provided by the second surgical instrument as the first surgical instrument is moved in a first direction, perform a curvature mitigating procedure to move the second surgical instrument in a second direction that is opposite to the first direction to mitigate the curvature.

2. The surgical instrument control device of claim 1,
wherein the processor is further configured to perform the curvature mitigating procedure by controlling the second driving part.

3. The surgical instrument control device of claim 1,
wherein positions or shapes of the first surgical instrument and the second surgical instrument are read using an angiogram.

4. The surgical instrument control device of claim 3,
wherein the processor is further configured to determine whether a curvature occurs in the path provided by the second surgical instrument by analyzing the angiogram.

5. The surgical instrument control device of claim 4,
wherein, when the curvature is determined to be mitigated by a reference value or more in the path provided by the second surgical instrument, the processor is further configured to stop the curvature mitigating procedure.

6. The surgical instrument control device of claim 1,
wherein the first surgical instrument is moved along the path provided by the second surgical instrument while surrounding the second surgical instrument from an outside.

7. The surgical instrument control device of claim 6,
wherein the first surgical instrument comprises one of a balloon catheter, a stent catheter, or a microcatheter, and
the second surgical instrument comprises a guide wire.

8. A surgical instrument control device comprising:
a first driving part comprising a first roller module and a second roller module disposed in parallel to grip a first surgical instrument therebetween;
a second driving part comprising a third roller module and a fourth roller module disposed in parallel to grip a second surgical instrument therebetween; and
a processor configured to control operations of the first driving part and the second driving part,
wherein the processor is further configured to:
control the first driving part to move the first surgical instrument forward and backward along a path provided by the second surgical instrument in response to an input user command, and
while the first surgical instrument is moved along the path provided by the second surgical instrument, when an end portion of the first surgical instrument is not moved in a first direction due to resistance applied to the end portion of the first surgical instrument, perform a path difference mitigating procedure to move the second surgical instrument in the first direction to mitigate a path difference between the first surgical instrument and the second surgical instrument.

9. The surgical instrument control device of claim 8,
wherein the processor is further configured to perform the path difference mitigating procedure by controlling the second driving part.

10. The surgical instrument control device of claim 8,
wherein an end portion of the second surgical instrument is disposed across an entrance of a blood vessel leading to a lesion.

11. The surgical instrument control device of claim 8,
wherein positions or shapes of the first surgical instrument and the second surgical instrument are read using an angiogram.

12. The surgical instrument control device of claim 11,
wherein the processor is further configured to analyze the angiogram to determine whether the end portion of the first surgical instrument is not moved in the first direction while a force to move the first surgical instrument is applied.

13. The surgical instrument control device of claim 12,
wherein, when the path difference between the first surgical instrument and the second surgical instrument is determined to be mitigated by a reference value or more, the processor is further configured to stop the path difference mitigating procedure.

14. The surgical instrument control device of claim 8,
wherein the first surgical instrument is moved inside the second surgical instrument along the path provided by the second surgical instrument.

15. The surgical instrument control device of claim 14,
wherein the first surgical instrument comprises one of a guide wire or a microcatheter, and
the second surgical instrument comprises a guide catheter.
